Europäisches Patentamt

European Patent Office

Office européen des brevets

(19)

(11) **EP 1 595 493 A1**

(12) **EUROPEAN PATENT APPLICATION**

(43) Date of publication:
**16.11.2005 Bulletin 2005/46**

(51) Int Cl.$^7$: **A61B 5/00**

(21) Application number: **04029345.8**

(22) Date of filing: **10.12.2004**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **10.05.2004 JP 2004140159**

(71) Applicant: **Hitachi, Ltd.**
**Tokyo 100-8280 (JP)**

(72) Inventors:
• **Cho, Ok-Kyung Hitachi, Ltd. Int. Prop.Group**
 **Chiyoda-ku Tokyo 100-8220 (JP)**

• **Kim, Yoon-Ok Hitachi, Ltd. Int. Prop.Group**
 **Chiyoda-ku Tokyo 100-8220 (JP)**
• **Nagata, Koji Hitachi, Ltd. Int. Prop.Group**
 **Chiyoda-ku Tokyo 100-8220 (JP)**
• **Mitsumaki, Hiroshi Hitachi, Ltd. Int. Prop.Group**
 **Chiyoda-ku Tokyo 100-8220 (JP)**

(74) Representative: **Strehl Schübel-Hopf & Partner**
 **Maximilianstrasse 54**
 **80538 München (DE)**

(54) **Blood sugar level measuring apparatus**

(57)    Blood sugar levels are measured non-invasively on the basis of temperature measurement. Measurement data is stabilized by correcting a non-invasive blood sugar level measurement value obtained by a temperature measuring system on the basis of the blood oxygen saturation and the blood flow volume.

# FIG. 1

ROOM TEMPERATURE (T$_r$)

BLOCK

HEAT DISSIPATION

T$_2$

HEAT TRANSFER

BODY-SURFACE TEMPERATURE (T$_s$)

T$_1$

BLOOD FLOW VOLUME

CAPILLARY BLOOD VESSEL

EP 1 595 493 A1

## EP 1 595 493 A1

**Description**

CLAIM OF PRIORITY

The present application claims priority from Japanese application JP 2002-140159 filed on May 10, 2004, the content of which is hereby incorporated by reference into this application.

BACKGROUND OF THE INVENTION

Field of the Invention

The present invention relates to a method and apparatus for non-invasive measurement of blood sugar levels for measuring glucose concentration in a living body without blood sampling.

Description of Related Art

Hilson *et al.* report facial and sublingual temperature changes in diabetics following intravenous glucose injection (Non-Patent Document 1). Scott *et al.* discuss the issue of diabetics and thermoregulation (Non-Patent Document 2). Based on the knowledge gained from such researches, Cho *et al.* suggest a method and apparatus for determining blood glucose concentration by temperature measurement without requiring the collection of a blood sample (Patent Documents 1 and 2).

Various other attempts have been made to determine glucose concentration without blood sampling. For example, a method has been suggested (Patent Document 3) whereby a measurement site is irradiated with near-infrared light of three wavelengths, and the intensity of transmitted light as well as the temperature of the living body is detected. A representative value of the second-order differentiated value of absorbance is then calculated, and the representative value is corrected in accordance with the difference between the living body temperature and a predetermined reference temperature. The blood sugar concentration corresponding to the thus corrected representative value is then determined. An apparatus is also provided (Patent Document 4) whereby a measurement site is heated or cooled while monitoring the living body temperature. The degree of attenuation of light based on light irradiation is measured at the moment of temperature change so that the glucose concentration responsible for the temperature-dependency of the degree of light attenuation can be measured. Further, an apparatus is reported (Patent Document 5) whereby an output ratio between reference light and transmitted light following the irradiation of the sample is taken, and then a glucose concentration is calculated in accordance with a linear expression of the logarithm of the output ratio and the living body temperature.

[Non-Patent Document 1] Diabete & Metabolisme, "Facial and sublingual temperature changes following intravenous glucose injection in diabetics" by R.M. Hilson and T.D.R. Hockaday, 1982, 8, 15-19
[Non-Patent Document 2] Can. J. Physiol. Pharmacol., "Diabetes mellitus and thermoregulation" by A.R. Scott, T. Bennett, I.A. MacDonald, 1987, 65, 1365-1376

[Patent Document 1] U.S. Patent No. 5,924,996
[Patent Document 2] U.S. Patent No. 5,795,305
[Patent Document 3] JP Patent Publication (Kokai) No. 2000-258343 A
[Patent Document 4] JP Patent Publication (Kokai) No. 10-33512 A (1998)
[Patent Document 5] JP Patent Publication (Kokai) No. 10-108857 A (1998)

SUMMARY OF THE INVENTION

Glucose (blood sugar) in blood is used for glucose oxidation reaction in cells to produce necessary energy for the maintenance of living bodies. In the basal metabolism state, in particular, most of the produced energy is converted into heat energy for the maintenance of body temperature. Thus, it can be expected that there is some relationship between blood glucose concentration and body temperature. However, as is evident from the way sicknesses cause fever, the body temperature also fluctuates due to factors other than blood glucose concentration. While methods have been proposed to determine blood glucose concentration by temperature measurement without blood sampling, they could hardly be considered sufficiently accurate.

It is an object of the invention to provide a method and apparatus for determining blood glucose concentration with high accuracy based on temperature data regarding a test subject without blood sampling.

Blood sugar is delivered to the cells throughout the human body via blood vessel systems, particularly the

2

capillary blood vessels. In the human body, complex metabolic pathways exist. Glucose oxidation is a reaction in which, fundamentally, blood sugar reacts with oxygen to produce water, carbon dioxide, and energy. Oxygen herein refers to the oxygen delivered to the cells via blood. The volume of oxygen supply is determined by the blood hemoglobin concentration, the hemoglobin oxygen saturation, and the volume of blood flow. On the other hand, the heat produced in the body by glucose oxidation is dissipated from the body by convection, heat radiation, conduction, and so on. On the assumption that the body temperature is determined by the balance between the amount of energy produced in the body by glucose burning, namely heat production, and heat dissipation such as mentioned above, the inventors set up the following model:

(1) The amount of heat production and the amount of heat dissipation are considered equal.
(2) The amount of heat production is a function of the blood glucose concentration and the volume of oxygen supply.
(3) The volume of oxygen supply is determined by the blood hemoglobin concentration, the blood hemoglobin oxygen saturation, and the volume of blood flow in the capillary blood vessels.
(4) The amount of heat dissipation is mainly determined by heat convection and heat radiation.

According to this model, we achieved the present invention after realizing that blood sugar levels can be accurately determined on the basis of the results of measuring the temperature of the body surface and parameters relating to the blood oxygen concentration and the blood flow volume. The parameters can be measured, e.g., from a part of the human body, such as the fingertip. The parameters relating to convection and radiation can be determined by measuring the temperature on the fingertip. The parameters relating to the blood hemoglobin concentration and the blood hemoglobin oxygen saturation can be determined by spectroscopically measuring blood hemoglobin and then finding the ratio between hemoglobin bound with oxygen and hemoglobin not bound with oxygen. With regard to the parameters relating to the blood hemoglobin concentration and blood hemoglobin oxygen saturation, instead of actually performing measurements, constants that are stored in advance may be used without adversely affecting the measurement accuracy. The parameter relating to the volume of blood flow can be determined by measuring the amount of heat transfer from the skin.
In one example, the invention provides a blood sugar level measuring apparatus comprising:

a heat amount measurement portion for measuring a plurality of temperatures deriving from a body surface and obtaining information used for calculating the amount of heat transferred by convection and the amount of heat transferred by radiation, both related to the dissipation of heat from said body surface;
an oxygen amount measuring portion for obtaining information about blood oxygen amount;
a memory portion for storing relationships between parameters corresponding to said plurality of temperatures and blood oxygen amount and blood sugar levels;
a calculating portion which converts a plurality of measurement values fed from said heat amount measuring portion and said oxygen amount measurement portion into said parameters, and calculates a blood sugar level by applying said parameters to said relationship stored in said memory portion; and
a display portion for displaying the blood sugar level calculated by said calculating portion,

wherein:

said oxygen amount measurement portion includes a blood flow volume measurement portion for obtaining information about blood flow volume, and an optical measurement portion for obtaining hemoglobin concentration and hemoglobin oxygen saturation in blood, wherein said blood flow volume measurement portion includes:

a body-surface contact portion;
an adjacent temperature detector disposed adjacent to said body-surface contact portion;
a heat-conducting member disposed adjacent to said body-surface contact portion; and
an indirect temperature detector for detecting the temperature at a position on said heat-conducting member that is spaced apart from said body-contact potion by 3.6 mm or more.

In another example, the invention provides a blood sugar level measuring apparatus comprising:

an ambient temperature measuring portion for measuring ambient temperature;
a body-surface contact portion to be brought into contact with a body surface;
an adjacent temperature detector disposed adjacent to said body-surface contact portion;
a radiation heat detector for measuring radiation heat from said body-surface;
a heat-conducting member disposed adjacent to said body-surface contact portion;

an indirect temperature detector disposed adjacent to said heat-conducting member at a position spaced apart from said body-surface contact portion by 3.6 mm or more, for detecting the temperature at the position spaced apart from said body-surface contact portion;

a light source for irradiating said body-surface contact portion with light of at least two different wavelengths;

a photodetector for detecting reflected light produced as said light of at least two different wavelengths is reflected on said body surface;

a calculating portion including a conversion portion for converting the outputs of said adjacent temperature detector, said indirect temperature detector, said ambient temperature measuring portion, said radiation heat detector, and said photodetector into parameters, and a processing portion in which relationships between said parameters and blood sugar levels are stored in advance, said processing portion calculating a blood sugar level by applying said parameters to said relationships; and

a display portion for displaying the blood sugar level outputted from said calculation portion.

In yet another example, the invention provides a blood sugar level measuring apparatus comprising:

an ambient temperature measuring portion for measuring ambient temperature;

a body-surface contact portion to be brought into contact with a body surface;

an adjacent temperature detector disposed adjacent to said body-surface contact portion;

a radiation heat detector for measuring radiation heat from said body surface;

a heat-conducting member disposed adjacent to said body-surface contact portion;

an indirect temperature detector disposed adjacent to said heat-conducting member at a position spaced apart from said body-surface contact portion by 3.6 mm or more, for detecting the temperature at the position spaced apart from said body-surface contact portion;

a memory portion in which information regarding blood hemoglobin concentration and hemoglobin oxygen saturation is stored;

a calculation portion including a conversion portion for converting the outputs of said adjacent temperature detector, said indirect temperature detector, said ambient temperature measuring portion, and said radiation heat detector, into a plurality of parameters, and a processing portion in which relationships between said parameters and blood sugar levels are stored, said calculation portion calculating a blood sugar level by applying said parameters to said relationships; and

a display portion for displaying the blood sugar level outputted from said calculation portion.

In accordance with the invention, a highly accurate apparatus and method for non-invasively measuring blood sugar levels can be provided.

BRIEF DESCRIPTION OF THE DRAWINGS

Fig. 1 shows a model of the transfer of heat from a body surface to a block.
Fig. 2 shows temporal changes in the measurement values of temperatures $T_1$ and $T_2$.
Fig. 3 shows an example of measurement of a temporal change in temperature $T_3$.
Fig. 4 shows the relationship between measurement values obtained by various sensors and parameters derived therefrom.
Fig. 5 illustrates dimensions, for example.
Fig. 6 shows the relationship between skin temperature and blood flow volume.
Fig. 7 shows the relationship between blood flow volume and the heat conductivity of skin.
Fig. 8 illustrates the process of heat conduction.
Fig. 9 shows the relationship between time and temperature permeation thickness.
Fig. 10 illustrates the temporal rate of change of temperature permeation thickness.
Fig. 11 shows a top plan view of a non-invasive blood sugar level measuring apparatus according to the present invention.
Fig. 12 shows an operation procedure for the apparatus.
Fig. 13 shows the details of a measurement portion.
Fig. 14 is a conceptual chart illustrating the flow of data processing in the apparatus.
Fig. 15 is a chart plotting the glucose concentration values calculated by the invention and the glucose concentration values measured by the enzyme electrode method.
Fig. 16 shows the details of another example of the measurement portion.

Fig. 17 is a conceptual chart showing data storage locations in the apparatus.

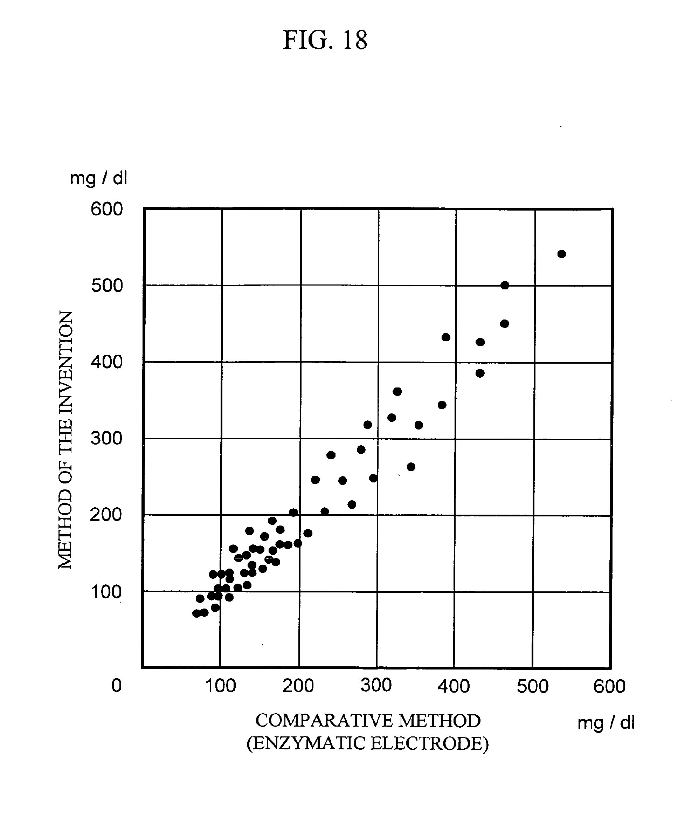

Fig. 18 is a chart plotting the glucose concentration values calculated by the invention and the glucose concentration values measured by the enzyme electrode method.

DETAILED DESCRIPTION OF THE PREFERRED EMBODIMENTS

The invention will now be described by way of preferred embodiments thereof with reference made to the drawings.

Initially, the above-mentioned model will be described in more specific terms. Regarding the amount of heat dissipation, convective heat transfer, which is one of the main causes of heat dissipation, is related to temperature difference between the ambient (room) temperature and the body-surface temperature. The amount of heat dissipation due to radiation, which is another main cause of dissipation, is proportional to the fourth power of the body-surface temperature according to the Stefan-Boltzmann law. Thus, it can be seen that the amount of heat dissipation from the human body is related to the room temperature and the body-surface temperature. On the other hand, the amount of oxygen supply, which is a major factor related to the amount of heat production, is expressed as the product of hemoglobin concentration, hemoglobin oxygen saturation, and blood flow volume.

The hemoglobin concentration can be measured from the absorbance at the wavelength at which the molar absorbance coefficient of the oxyhemoglobin is equal to that of the reduced (deoxy-) hemoglobin (equal-absorbance wavelength). The hemoglobin oxygen saturation can be measured by measuring the absorbance at the equal-absorbance wavelength and the absorbance at at least one different wavelength at which the ratio between the molar absorbance coefficient of the oxyhemoglobin and that of the reduced (deoxy-) hemoglobin is known, and then solving simultaneous equations. Namely, the hemoglobin concentration and hemoglobin oxygen saturation can be obtained by conducting the measurement of absorbance at at least two wavelengths.

The rest is the blood flow volume, which can be measured by various methods. One example will be described below.

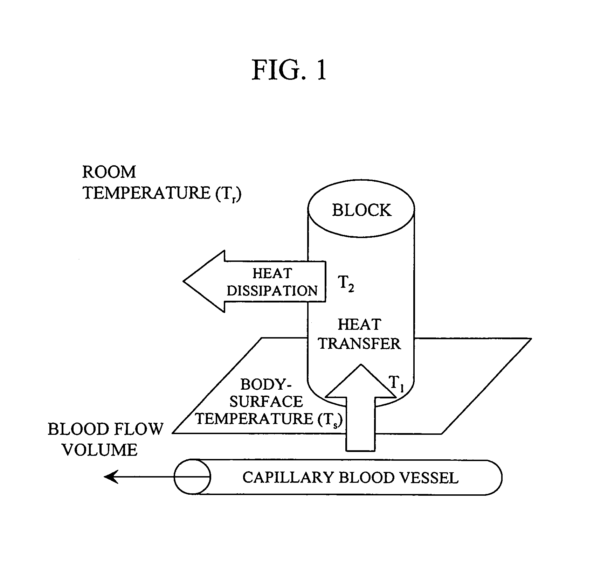

Fig. 1 shows a model for the description of the transfer of heat from the body surface to a solid block having a certain heat capacity when the block is brought into contact with the body surface for a certain time and then separated. The block is made of resin such as plastic or vinyl chloride. In the illustrated example, attention will be focused on the temporal variation of the temperature $T_1$ of a portion of the block that is brought into contact with the body surface, and the temporal variation of the temperature $T_2$ at a point on the block spaced apart from the body surface. The blood flow volume can be estimated by monitoring mainly the temporal variation of the temperature $T_2$ (at the spatially separated point on the block). The details will follow.

Before the block comes into contact with the body surface, the temperatures $T_1$ and $T_2$ at the two points of the block are equal to the room temperature $T_r$. When a body-surface temperature $T_s$ is higher than the room temperature $T_r$ as the block comes into contact with the body surface, the temperature $T_1$ swiftly rises due to the transfer of heat from the skin, and it approaches the body-surface temperature $T_s$. On the other hand, the temperature $T_2$ is lowered from the temperature $T_1$ as the heat conducted through the block is dissipated from the block surface, and it rises more gradually. The temporal variation of the temperatures $T_1$ and $T_2$ depends on the amount of heat transferred from the body surface to the block, which in turn depends on the blood flow volume in the capillary blood vessels under the skin. If the capillary blood vessels are regarded as a heat exchanger, the coefficient of transfer of heat from the capillary blood vessels to the surrounding cell tissues is given as a function of the blood flow volume. Thus, by measuring the amount of heat transfer from the body surface to the block by monitoring the temporal variation of the temperatures $T_1$ and $T_2$, the amount of heat transferred from the capillary blood vessels to the cell tissues can be estimated. Based on this estimation, the blood flow volume can then be estimated. Thus, by tracking the temperature change in $T_1$ and $T_2$ over time and thereby measuring the amount of heat transferred from the body surface to the block, the heat transfer amount from the capillary blood vessels to the cell tissue can be estimated, which in turn allows for the estimation of the blood flow volume.

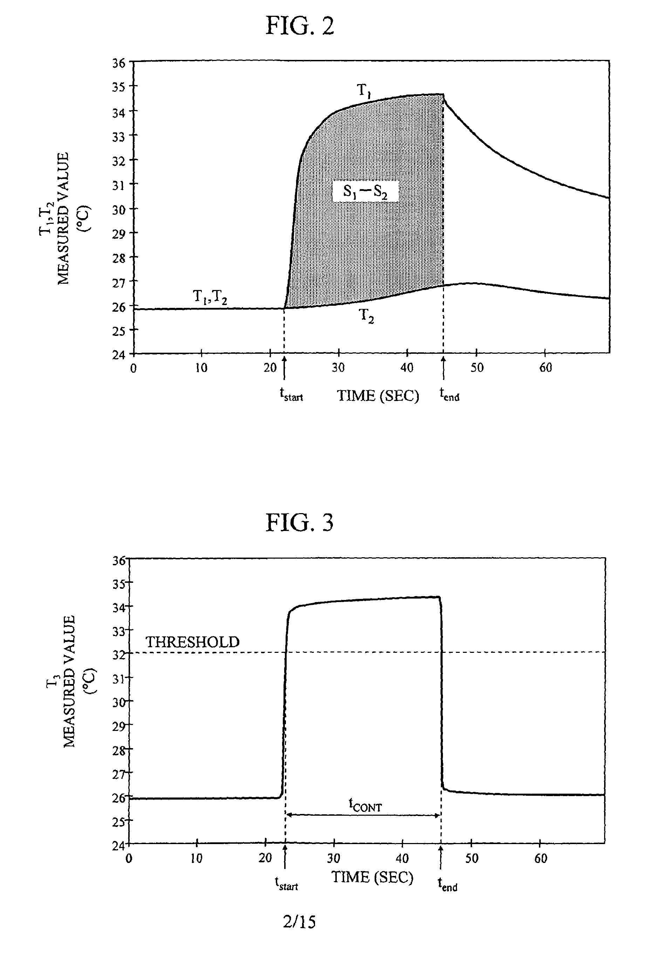

Fig. 2 shows the temporal variation of the measured values of the temperature $T_1$ at the portion of the block in contact with the body surface and the temperature $T_2$ at the position on the block spaced apart from the body-surface contact position. As the block comes into contact with the body surface, the $T_1$ measured value swiftly rises, and it gradually drops as the block is brought out of contact.

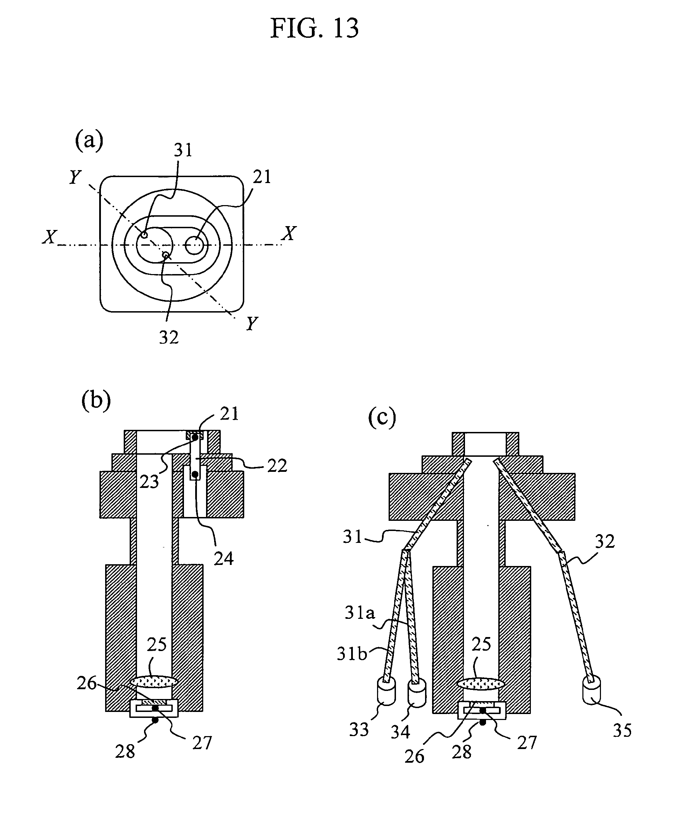

Fig. 3 shows the temporal variation of the value of the temperature $T_3$ measured by a radiation-temperature detector. As the detector detects the temperature $T_3$ that is due to radiation from the body surface, it is more sensitive to temperature changes than other sensors. Because radiation heat propagates as an electromagnetic wave, it can transmit temperature changes instantaneously. Thus, by locating the radiation-temperature detector near where the block contacts the body surface so as to detect radiated heat from the body surface, as shown in Fig. 13 (which will be described later), the time of start of contact $t_{start}$ and the time of end of contact $t_{end}$ between the block and the body surface can be detected from changes in the temperature $T_3$. For example, a temperature threshold value is set as shown in Fig. 3. The contact start time $t_{start}$ is when the temperature threshold value is exceeded. The contact end time tend is when the temperature $T_3$ drops below the threshold. The temperature threshold is set at 32°C, for example.

Then, the $T_1$ measured value between $t_{start}$ and tend is approximated by an S curve, such as a logistic curve. A logistic curve is expressed by the following equation:

$$T = \frac{b}{1+c \times \exp(-a \times t)}$$

where T is temperature, and t is time.

The measured value can be approximated by determining coefficients a, b, c, and d using the non-linear least-squares method. For the resultant approximate expression, T is integrated between time $t_{start}$ and time tend to obtain a value $S_1$.

Similarly, an integrated value $S_2$ is calculated from the $T_2$ measured value. The smaller $(S_1 - S_2)$ is, the larger the amount of transfer of heat is from the finger surface to the position of $T_2$. $(S_1 - S_2)$ becomes larger with increasing finger-surface contact time $t_{CONT}$ (=$t_{end}$ - $t_{start}$). Thus, $a_5/(t_{CONT} \times (S_1 - S_2))$ is designated as a parameter $X_5$ indicating the volume of blood flow, using $a_5$ as a proportionality coefficient.

It will be seen from the above discussion that the measured amounts necessary for the determination of blood glucose concentration by the above-described model are the room temperature (ambient temperature), body surface temperature, temperature changes in the block brought into contact with the body surface, the temperature due to radiation from the body surface, and absorbance at at least two wavelengths.

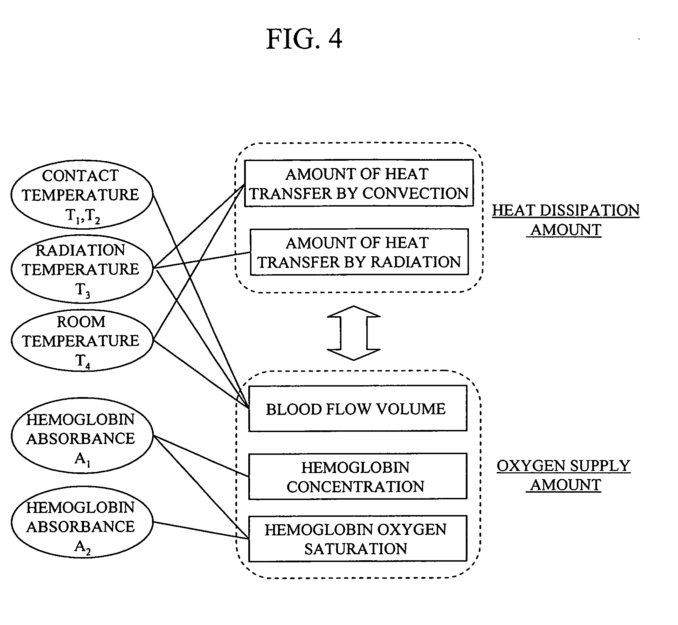

Fig. 4 shows the relationships between the measured values provided by various sensors and the parameters derived therefrom. A block is brought into contact with the body surface, and chronological changes in two kinds of temperatures $T_1$ and $T_2$ are measured by two temperature sensors provided at two locations of the block. Separately, radiation temperature $T_3$ on the body surface and room temperature $T_4$ are measured. Absorbance $A_1$ and $A_2$ are measured at at least two wavelengths related to the absorption of hemoglobin. The temperatures $T_1$, $T_2$, $T_3$, and $T_4$ provide parameters related to the volume of blood flow. The temperature $T_3$ provides a parameter related to the amount of heat transferred by radiation. The temperatures $T_3$ and $T_4$ provide parameters related to the amount of heat transferred by convection. The absorbance $A_1$ provides a parameter related to the hemoglobin concentration, and absorbance $A_1$ and $A_2$ provide parameters related to the hemoglobin oxygen saturation.

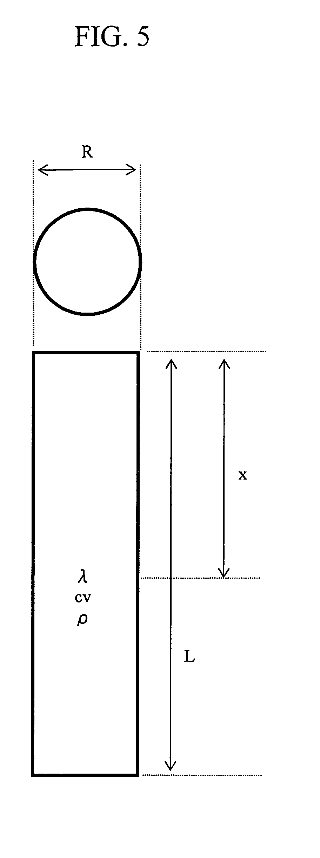

Fig. 5 schematically shows a block used in the invention. The block is columnar in shape and has a diameter R and a length L. As will be seen from the above discussion, the size of the block (length L (m), diameter R (m)), which is provided for the estimation of whether the blood flow volume shown in Fig. 1 is large or small, and thermal characteristics, such as the heat conductivity $\lambda$ (J/s·m·K) and the heat capacity U (J/K: specific heat capacity cv (J/K·kg) $\times$ block density $\rho$ (kg/m$^3$) $\times$ block volume V (m$^3$)), for example, are obviously important factors that determine measurement accuracy. The heat conductivity $\lambda$ is a value specific to the substance as the material of the block, and it indicates how easily heat is transferred. The heat capacity U indicates how much the temperature of the block changes due to the heat supplied thereto. Another factor that determines measurement accuracy, in addition to the heat characteristics of the block, is the distance (m) of a position x at which measurement is conducted from the point of contact between the block and a heat source.

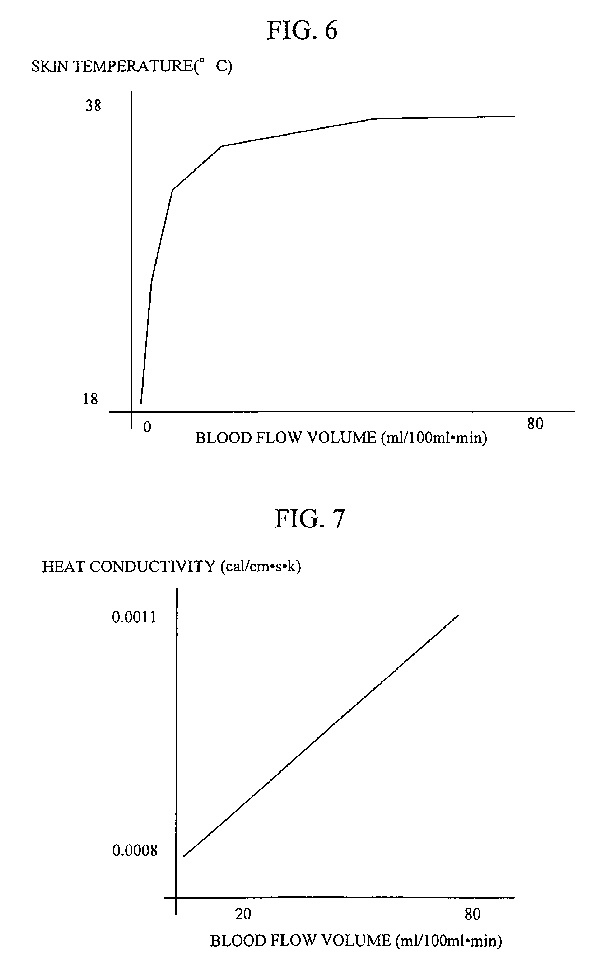

Fig. 6 shows the relationship between the blood flow volume in the finger and the skin temperature on the finger. Fig. 7 shows the relationship between the blood flow volume in the skin of the finger and the heat conductivity of the finger skin. As shown by these figures, the blood flow volume in the finger can be known by measuring the finger skin temperature or the finger skin heat conductivity. Of these relationships, the blood flow volume in the finger is directly related to the heat conductivity of the finger skin. The finger skin temperature is determined by the finger skin heat conductivity that is determined by the finger blood flow volume, the temperature inside the finger, and the ambient temperature. Namely, since the internal temperature of peripheral parts, such as a finger, varies depending on the environment, the temperature inside the finger may differ so that the blood flow volume may differ even if the finger skin temperature is the same. Therefore, in order to correctly measure the blood flow volume in the finger skin, the heat conductivity of the finger skin must be measured.

When a person is at rest, the heat flux M (J/s·m$^2$) generated by heat production, the heat flux C (J/s·m$^2$) dissipated from the skin by heat transfer, and the heat flux R (J/s·m$^2$) dissipated by radiation satisfy the following relationship:

$$M=C+R$$

such that they are in a state of thermal equilibrium.

Other factors that exist in reality, such as the dissipation of heat accompanying the evaporation of water on the body surface, are ignored herein.

In the state of thermal equilibrium where the above equation holds, the heat conduction from inside the skin to the skin surface is in a state of steady heat conduction, where the following equation holds:

$$M=\lambda 0((T0-Ts)/L)$$

where $\lambda 0$ is the heat conductivity of the finger skin, T0 is the internal temperature of the finger, Ts is the finger skin surface temperature, and L is the thickness of the finger skin. Since the skin thickness is generally constant, it is assumed to be a known value herein. The heat flux M due to heat production can be known by measuring the heat flux C $(J/s \cdot m^2)$ dissipated from the skin by heat transfer and the heat flux R $(J/s \cdot m^2)$ dissipated by radiation. However, in reality, the heat transfer coefficient h $(J/s \cdot m^2 \cdot K)$ of air, which is a parameter necessary for determining the heat flux C $(J/s \cdot m^2)$ dissipated by heat transfer, differs greatly depending on the state of the flow of air. Specifically, within the range of air flow from natural convection to nearly forced convection,

$$1 < h(J/s \cdot m^2 \cdot K) < 300$$

so that it is difficult to accurately measure the heat flux dissipated by heat transfer. For this reason, the heat flux M due to heat production is measured by bringing a block into contact with the finger skin surface, as shown in Fig. 1.

An example of the method of measuring the heat flux M due to heat production based on contact with the block is described. In this example, the size and thermal characteristics of the block must not be such that the state of steady heat conduction from the inside of the skin to the skin surface is significantly broken upon contact of the block with the finger surface. Namely, the heat flux q from the finger skin surface to the block upon contact must satisfy q ≈ M. In order to satisfy this condition, the following relationship must be met.

When an object A is brought into contact with another object B with a certain temperature, the heat flux that flows via the contact surfaces of the both objects is preserved across the boundary. In such a short time interval that the contacting object A can be regarded as a semi-infinite object, the heat flux is expressed as follows:

$$q = \lambda 1 (Ts-Tr)/ \sqrt{(\pi \alpha t)}$$

where Ts is the surface temperature of object B, Tr is the ambient temperature and the initial temperature of object A at the opposite end to the contact surface thereof, $\lambda 1$ is the heat conductivity of object A, $\alpha$ is the thermometric conductivity of object A, and t is time.

Thus, the above-described condition is expressed by

$$M = C + R = q = \lambda 1 (Ts - Tr)/ \sqrt{(\pi \alpha t)}$$

With regard to the dissipation of heat from a human body, the amount of heat flux C $(J/s \cdot m^2)$ dissipated from the skin by heat transfer is substantially equal to the amount of heat flux R $(J/s \cdot m^2)$ dissipated by radiation, at approximately 20 to 30 $(J/s \cdot m^2)$. Therefore, the above-mentioned condition that the state of steady heat conduction from the inside of the skin to the skin surface must not be significantly broken upon contact of the block with the finger surface can be roughly satisfied by specifying the thermal characteristics of the block such that the heat conduction produced upon contact with the block is approximately twice as much as the amount of heat transfer from the human body to air. Accordingly, using the relationship

$$h \cdot t = 2\lambda \sqrt{(t/\pi/\alpha)}$$

that states that the quantity of heat that passes through a unit area in a contact time t(s) for measurement is equal, the characteristics of the block, namely the heat conductivity $\lambda$ (J/s.m.K), the specific heat capacity cv (J/K·kg), and the block density $\rho$ (kg/m³), are specified such that, in the case where t = 10 s, for example:

$$3 < \sqrt{(\lambda \cdot cv \cdot \rho)} < 900 \quad \text{Condition 1 (when measurement time is 10 s)}$$

Examples of the substance that satisfy condition 1 include most general resins among the resin materials, such as polyvinylchloride and ABS resin (a resin made of acrylonitrile (A), butadiene (B), and styrene (S)). The general values of the characteristics of ABS are such that the heat conductivity $\lambda$ = 0.2 (J/s.m.K), the specific heat volume cv = 1600 (J/K·kg), and the block density $\rho$ = 1060 (kg/m$^3$). Thus, when measurement time t = 10 s,

$$\sqrt{(\lambda \cdot cv \cdot \rho)} = 582$$

which satisfies condition 1. The general values of the characteristics of polyvinylchloride are such that the heat conductivity $\lambda$ = 0.17 (J/s·m·K), the specific heat volume cv = 1640 (J/K·kg), and the block density $\rho$ = 1390 (kg/m$^3$). Thus, when measurement time t = 10 s,

$$\sqrt{(\lambda \cdot cv \cdot \rho)} = 622$$

thus satisfying condition 1. Generally, the substances that satisfy condition 1 are resins. It is desirable that the heat conductivity of the substance used is in the range of from about 0.1 J/s·m·K to about 0.3 J/s.m.K.

In the above-described method of measuring blood sugar levels, the block is brought into contact with the finger skin surface, and temperatures are measured at two points. Such a problem of determining the thermal boundary condition from a temperature distribution is generalized as an inverse problem. A typical example of the approximate analysis for this inverse problem is the profile method. In the profile method, there is a necessary condition that the object of calculation can be treated as a semi-infinite object. A certain object can be handled as a semi-infinite object within a range of temperature distribution that is produced by heat conduction within a short time interval, and such a range is defined as a temperature permeation thickness ($\delta$(m)). Namely, if the measurement point is located at the terminal point of the temperature permeation thickness that has spread within a specified measurement time, a solution to the inverse problem can be calculated using the measurement value obtained at the terminal point, and then the thermal boundary condition can be determined. The temperature permeation thickness is generally the distance between a contact surface and a point where a temperature change of 1% of the surface temperature is produced. In the case of the invention, where the object of measurement is a human body, the temperature at the contact portion is approximately 30°C, of which 1% is approximately 0.3°C. In this case, the temperature permeation thickness is given, according to a strict solution to unsteady conduction, by

$$\delta = 3.6 \sqrt{((t \cdot \lambda)/(cv \cdot \rho))}$$

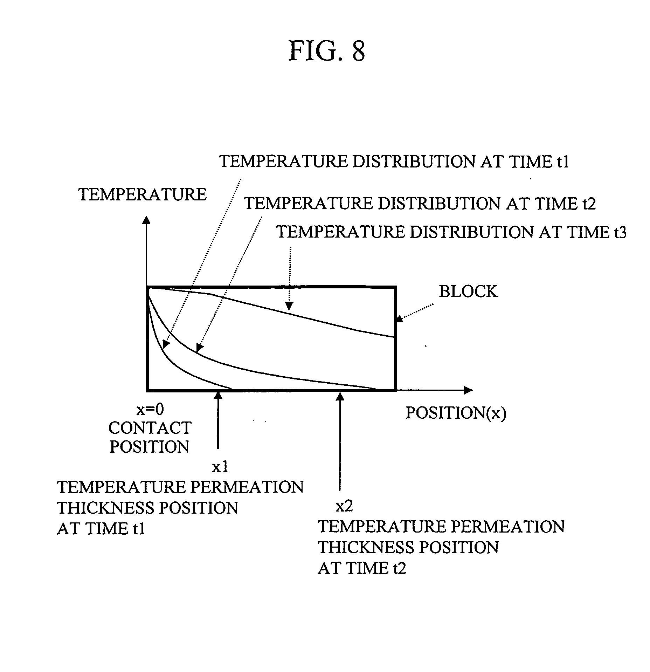

Fig. 8 schematically shows changes in temperature distribution after the block is in contact with the heat source. The temperature distribution spreads over time from the position of contact, eventually covering the entire block. In the distribution at time t1 in the figure, the temperature permeation thickness is sufficiently smaller than the length of the block. In this state, the temperature distribution is not affected by the length of the block, so that the block can be handled as a semi-infinite object. Similarly, at time t2, the temperature permeation thickness is still sufficiently smaller than the block length and the temperature distribution is not yet affected by the block length, enabling the block to be handled as a semi-infinite object. However, at time t3, the temperature permeation thickness is larger than the block length, resulting in a shape of the temperature distribution that is affected by the block length. In this state, the measurement method employing a block is not valid anymore. Thus, the method based on the use of a block must employ a block with a length larger than the temperature permeation thickness.

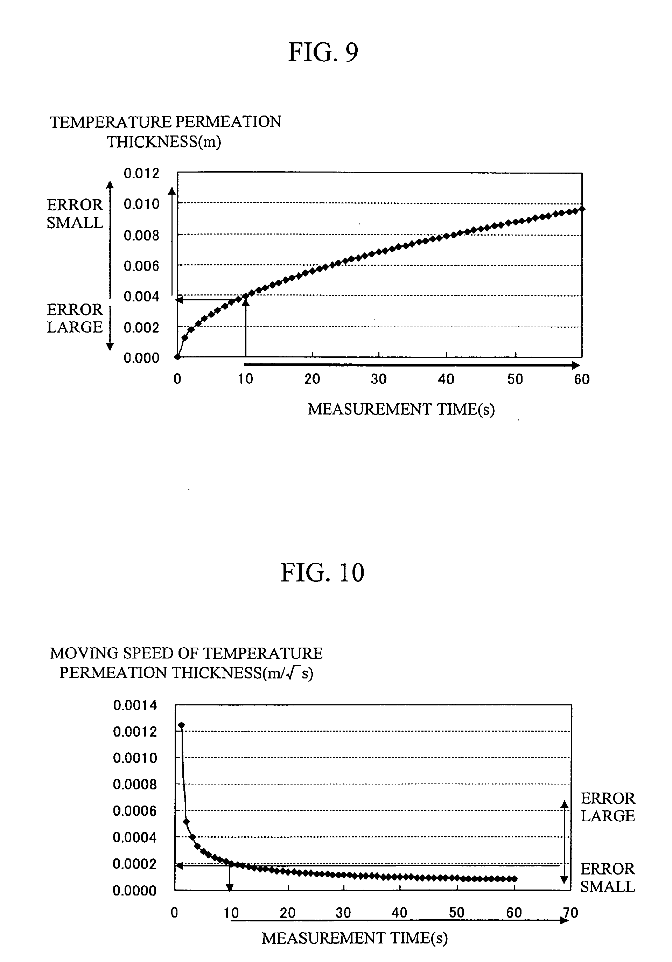

As described above, the temperature permeation thickness is dependent on the physical properties of the block and the contact time with the heat source (measurement time). Therefore, once the material of the block is determined, the valid time of measurement using the block and the necessary length of the block are specified by the material. Fig. 9 shows the relationship between measurement time t and temperature permeation thickness $\delta$ for a material such as polyvinylchloride or ABS. Specifically, the relationship indicates a minimum block length L required for the method of measurement using a block to be valid at measurement time t in the case where polyvinylchloride or ABS is used.

Fig. 10 shows a temporal rate of change of the temperature permeation thickness shown in Fig. 9, which is dependent on the thermometric conductivity (m$^2$/s).

As will be seen from this figure, the temporal rate of change of temperature permeation thickness is large when the measurement time is 10 s or less, where a sharp temperature change is indicated to occur. On the other hand, when the measurement time is 10 s or longer, the temporal rate of change of temperature permeation thickness is substantially constant. This indicates that the influence of variation in measurement time (which is caused by the measurement flow in which the examined subject first confirms a message generated by the measurement equipment and then performs an operation accordingly) greatly differs at the 10-s measurement-time boundary. Thus, a region where a T2 temperature measurement error due to variation in measurement time is large and another region with a small T2 temperature measurement error can be defined, as shown in Figs. 10 and 9. It is therefore preferable to set the measurement time to be 10 s or longer in order to minimize the deterioration in measurement accuracy due to variation in measurement time.

Now that it has been shown that the measurement time should preferably be not less than 10 s from the viewpoint of measurement accuracy, the minimum block length for obtaining accurate measurement results in a measurement using a block is determined. Namely, the minimum block length is, from Fig. 9, the temperature permeation thickness when the measurement time (duration of contact with the heat source) is 10 s. Therefore, the length L of the block must satisfy the following condition:

$$L > \delta \qquad \qquad \text{Condition 2}$$

With regard to the measurement point x, the point at which the temperature of the block is measured is disposed at a point spaced apart from the contact point by a distance substantially corresponding to the temperature permeation thickness that has been determined in accordance with the aforementioned expression of temperature permeation thickness upon specifying the measurement time. Thus, measurement position x must satisfy the following condition:

$$x > \delta \qquad \qquad \text{Condition 3}$$

Conditions 2 and 3 should be satisfied simultaneously with condition 1. When the measurement time is 10 s, namely the lower-limit value, and when the physical properties of an ABS resin (heat conductivity $\lambda = 0.2$ (J/s.m.K), specific heat capacity cv = 1600 (J/K·kg), and block density $\rho$ = 1060 (kg/m$^3$)) are used in condition 1, the temperature permeation thickness ($\delta$(m)) turns out to be approximately 3.6 mm. A similar value is obtained for polyvinylchloride. Thus, the condition (condition 2) to be satisfied by length L of the block is such that

$$L > 3.6 \text{ mm}$$

The condition (condition 3) required of the temperature measurement point is

$$x > 3.6 \text{ mm}$$

Thus far, the thermal characteristics and length required of the contacted block by the measurement principle, as well as the temperature measurement position, have been specified. In addition, similar requirements are set forth for the area of contact between the block and the finger. The area of contact between the block and the finger corresponds to the cross-sectional area of the block. The most fundamental requirement that specifies the block cross-sectional area concerns the size (width) of the finger, which is on the order of 10 to 15 mm. Thus, as a condition for allowing the block to be in contact with the finger with high reproducibility at all times, it is required that the diameter (R (m)) of the contact portion of the block be not larger than one half the width of the finger.

Accordingly, the diameter of the contact portion of the block must satisfy the following condition:

$$R < 7.5 \text{ mm} \qquad \qquad \text{Condition 4}$$

By bringing the block satisfying the aforementioned conditions into contact with the skin, measuring a temperature change at two points, and then solving the inverse problem, the heat flux qx that has actually flowed to the block can be calculated. From this measurement, a relationship expressed by the following equation is obtained:

$$M = \lambda 0((T0 - Ts)/L) = qx$$

By solving as simultaneous equations the above value and an expression containing $\lambda$0 and T0 that is obtained when the block is assumed to be a concentrated heat capacity and modeled based on an electric-circuit analogy, the heat conductivity λ0 of the skin that is to be determined can be calculated. From this value, information regarding the blood flow volume can be obtained using the relationship shown in Fig. 7.

Hereafter, an example of an apparatus for non-invasively measuring blood sugar levels according to the principle of the invention will be described.

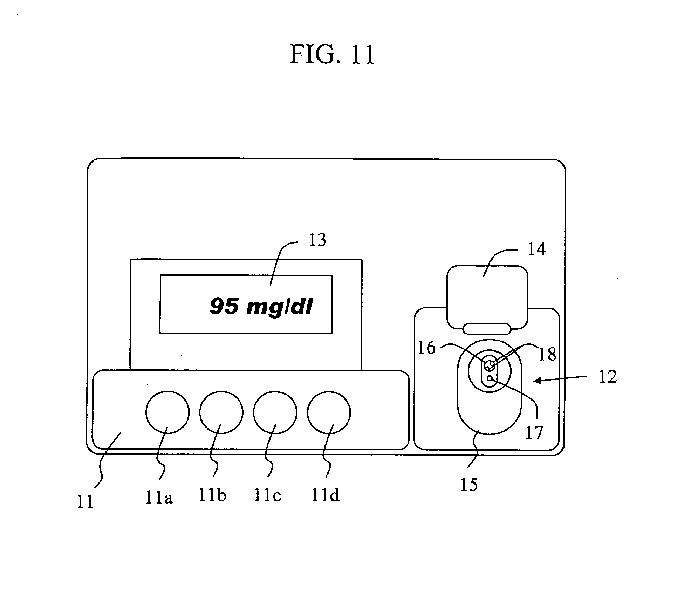

Fig. 11 shows a top plan view of a non-invasive blood sugar level measuring apparatus according to the invention. While in this example the skin on the ball of the fingertip is used as the body surface, other parts of the body surface may be used.

On the top surface of the apparatus are provided an operating portion 11, a measuring portion 12 where the finger to be measured is to be placed, and a display portion 13 for displaying measurement results, the state of the apparatus, measured values, for example. The operating portion 11 includes four push buttons 11a to 11d for operating the apparatus. The measuring portion 12 has a cover 14 which, when opened (as shown), reveals a finger rest portion 15 with an oval periphery. The finger rest portion 15 accommodates an opening end 16 of a radiation-temperature sensor portion, a contact-temperature sensor portion 17, and an optical sensor portion 18.

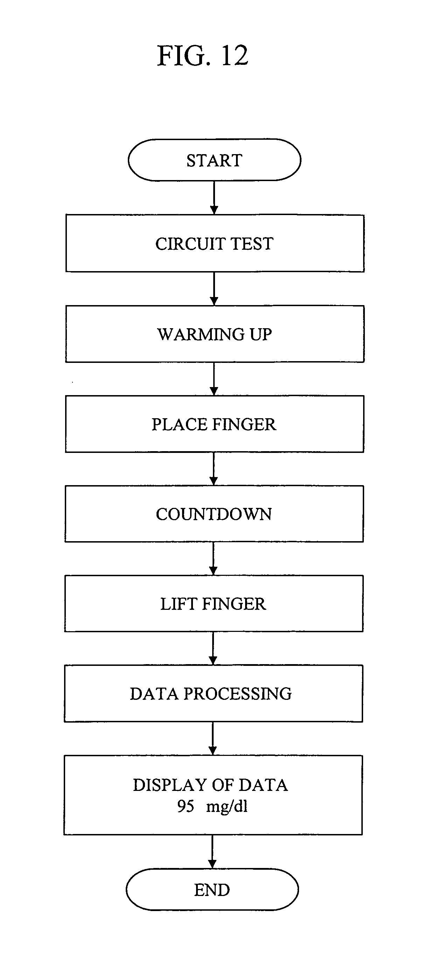

Fig. 12 shows a procedure for operating the apparatus. As a power button on the operating portion is pressed to turn on the apparatus, an indication "WARMING UP" is displayed on the LCD and the electronic circuits in the apparatus are warmed up. At the same time, a check program is activated to automatically check the electronic circuits. After the warm-up phase is finished, an indication "PLACE FINGER" appears on the LCD. As the user places his or her finger on the finger rest portion, a countdown is displayed on the LCD. When the countdown is over, an indication "LIFT FINGER" appears on the LCD. As the user puts his or her finger away, the LCD indicates "PROCESSING DATA." Thereafter, the display shows a blood sugar level, which is then stored in an IC card together with the date and time. After the user reads the displayed blood sugar level, he or she pushes a particular button on the operating portion. About one minute later, the apparatus displays a message "PLACE FINGER" on the LCD, thus indicating that the apparatus is ready for the next cycle of measurement.

Fig. 13 shows the measuring portion in detail. In Fig. 13, (a) is a top plan view, (b) is a cross section taken along line X-X of (a), and (c) is a cross section taken along line Y-Y of (a).

First, the process of measuring temperatures by the non-invasive blood sugar level measuring apparatus according to the invention will be described. In a portion of the measuring portion with which the examined portion (ball of the finger) is to come into contact, a thin plate 21 of a highly heat-conductive material, such as gold, is placed. A bar-shaped heat-conductive member 22, which is made of a material with a heat conductivity lower than that of the plate 21, such as polyvinylchloride, is thermally connected to the plate 21 and extends into the apparatus. The temperature sensors include a thermistor 23 that is an adjacent-temperature detector with respect to the examined portion for measuring the temperature of the plate 21, and a thermistor 24 that is an indirect-temperature detector with respect to the examined portion for measuring the temperature of a portion of the heat-conducting member which is spaced apart from the plate 21 by a certain distance. An infrared lens 25 is disposed inside the apparatus at such a position that the examined portion (ball of the finger) placed on the finger rest portion 15 can be seen through the lens. Below the infrared lens 25 is disposed a pyroelectric detector 27 via an infrared radiation-transmitting window 26. Another thermistor 28 is disposed in close proximity to the pyroelectric detector 27.

The heat-conducting member may be made of a material with a heat conductivity within the range of from 0.1 J/s·m·K to 0.3 J/2·m·K. In the present example, an ABS resin with a heat conductivity of 0.2 J/s.m.K was used as the heat-conducting member. Instead of ABS resins, polyvinylchloride may be used. The shape of the heat-conducting member is not particularly limited as long as it has a length of 3.6 mm or larger and a diameter of 7.5 mm or larger. In the present example, a column with a diameter of 2 mm and a length of 8 mm was used. The thermistor 24 is only required to be able to detect the temperature at a position spaced apart from the plate 21 by 3.6 mm or more. In the present example, the thermistor 24 was disposed so as to measure the temperature at a portion of the heat-conducting member that is spaced apart from the plate by 5 mm.

Thus, the temperature sensor portion of the measuring portion has four temperature sensors, and they measure four kinds of temperatures as follows:

(1) Temperature on the finger surface (thermistor 23): $T_1$
(2) Temperature of the heat-conducting member (thermistor 24): $T_2$
(3) Temperature of radiation from the finger (pyroelectric detector 27): $T_3$
(4) Room temperature (thermistor 28): $T_4$

The optical sensor portion 18 is described hereafter. The optical sensor portion 18 measures the hemoglobin concentration and the hemoglobin oxygen saturation necessary for the determination of the oxygen supply volume. In order to measure the hemoglobin concentration and the hemoglobin oxygen saturation, it is necessary to measure absorbance at at least two wavelengths. Fig. 13(c) shows an exemplary configuration for carrying out a two-wavelength measurement using two light sources 33 and 34 and a single detector 35.

The ends of two optical fibers 31 and 32 are located in the optical sensor portion 18. The optical fiber 31 is for optical irradiation, while the optical fiber 32 is for receiving light. As shown in Fig. 13(c), the optical fiber 31 connects to branch optical fibers 31 a and 31b, and the ends thereof are provided with light-emitting diodes 33 and 34 of two wavelengths. The receiving optical fiber 32 is provided at the end thereof with a photodiode 35. The light-emitting diode 33 emits light with a wavelength of 810 nm, while the light-emitting diode 34 emits light with a wavelength of 950 nm. The wavelength 810 nm is the equal-absorbance wavelength at which the molar absorbance coefficient of the oxyhemoglobin is equal to that of the reduced (deoxy-) hemoglobin. The wavelength 950 nm is the wavelength at which the difference between the molar absorbance coefficient of the oxyhemoglobin and that of the reduced hemoglobin is large.

The two light-emitting diodes 33 and 34 emit light in a time-sharing manner. The finger of an examined subject is irradiated with the light emitted by the light-emitting diodes 33 and 34 via the light-irradiating optical fiber 31. The light shone on the finger is reflected by the skin of the finger and is then incident on the light-receiving optical fiber 32, via which the light is detected by the photodiode 35. When the light with which the finger is irradiated is reflected by the skin of the finger, part of the light penetrates into the tissue through the skin and is absorbed by the hemoglobin in the blood that flows in capillary blood vessels. The measurement data provided by the photodiode 35 is reflectance R. Absorbance can be approximately calculated from $\log(1/R)$. Irradiation is conducted with light of wavelengths 810 nm and 950 nm, R is measured for each, and then $\log(1/R)$ is obtained, thereby measuring absorbance $A_1$ for wavelength 810 nm and absorbance $A_2$ for wavelength 950 nm.

When the reduced hemoglobin concentration is [Hb] and the oxyhemoglobin concentration is [HbO$_2$], absorbance $A_1$ and absorbance $A_2$ are expressed by the following equations:

$$A_1 = a \times ([Hb] \times A_{Hb}(810nm) + [HbO_2] \times A_{HbO_2}(810nm))$$

$$= a \times ([Hb] + [HbO_2]) \times A_{HbO_2}(810nm)$$

$$A_2 = a \times ([Hb] \times A_{Hb}(950nm) + [HbO_2] \times A_{HbO_2}(950nm))$$

$$= a \times ([Hb] + [HbO_2]) \times ((1 - \frac{[HbO_2]}{[Hb] + [HbO_2]}) \times A_{Hb}(950nm) + \frac{[HbO_2]}{[Hb] + [HbO_2]} \times A_{HbO_2}(950nm))$$

where $A_{Hb}$(810 nm) and $A_{Hb}$(950 nm), and $A_{Hb02}$(810 nm) and $A_{Hb02}$(950 nm) are the molar absorbance coefficients of the reduced hemoglobin and the oxyhemoglobin, respectively, and are known at the respective wavelengths. The term a is a proportionality coefficient. From the above equations, the blood hemoglobin concentration $([Hb]+[HbO_2])_T$ inside tissue and the blood hemoglobin oxygen saturation $([HbO_2]/([Hb]+[HbO_2]))_T$ are determined as follows:

$$[Hb] + [HbO_2] = \frac{A_1}{\alpha \times A_{HbO_2}(810nm)}$$

$$\frac{[HbO_2]}{[Hb] + [HbO_2]} = \frac{A_2 \times A_{HbO_2}(810nm) - A_1 \times A_{Hb}(950nm))}{A_1 \times (A_{HbO_2}(950nm) - A_{Hb}(950nm))}$$

While the above example involved the measurement of the hemoglobin concentration and hemoglobin oxygen saturation based on the measurement of absorbance at two wavelengths, it is also possible to reduce the influence of interfering components and increase measurement accuracy by measuring absorbance at three or more wavelengths.

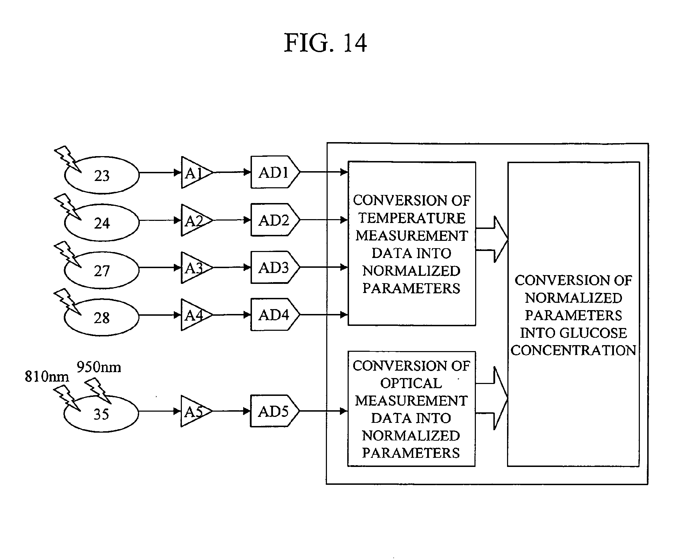

Fig. 14 is a conceptual chart showing the flow of data processing in the apparatus. The apparatus according to the present example is equipped with five sensors, namely a thermistor 23, a thermistor 24, a pyroelectric detector 27, a thermistor 28, and a photodiode 35. The photodiode 35 measures absorbance at wavelengths 810 nm and 950 nm. Thus, the apparatus is supplied with six kinds of measurement values.

The five kinds of analog signals are supplied via individual amplifiers A1 to A5 to analog/digital converters AD1 to AD5, where they are converted into digital signals. Based on the digitally converted values, parameters $x_i$ (i=1, 2, 3, 4, 5) are calculated. The following are specific descriptions of $x_i$ (where $a_1$ to $a_5$ are proportionality coefficients):

Parameter proportional to heat radiation

$$x_1 = \alpha_1 \times (T_3)^4$$

Parameter proportional to heat convection

$$x_2 = \alpha_2 \times (T_4 - T_3)$$

Parameter proportional to hemoglobin concentration

$$x_3 = a_3 \left( \frac{A_1}{a \times A_{HbO_2}(810nm)} \right)$$

Parameter proportional to hemoglobin saturation

$$x_4 = a_4 \times \left( \frac{A_2 \times A_{HbO_2}(810nm) - A_1 \times A_{Hb}(950nm))}{A_1 \times (A_{HbO_2}(950nm) - A_{Hb}(950nm))} \right)$$

Parameter proportional to blood flow volume

$$x_5 = a_5 \times \left( \frac{1}{t_{CONT} \times (S_1 - S_2)} \right)$$

Then, normalized parameters are calculated from mean values and standard deviations of parameters $x_i$ obtained from actual data on large numbers of able-bodied people and diabetic patients. A normalized parameter $X_i$ (where i=1, 2, 3, 4, 5) is calculated from each parameter $x_i$ according to the following equation:

$$X_i = \frac{x_i - \bar{x}_i}{SD(x_i)}$$

where

$x_i$: parameter
$\bar{x}_i$: mean value of the parameter
$SD(x_i)$: standard deviation of the parameter

Calculations are conducted to convert the above five normalized parameters into a glucose concentration to be eventually displayed. A program necessary for the calculations is stored in the ROM built inside the microprocessor in the apparatus. A memory area necessary for the calculations is ensured in a RAM similarly built inside the apparatus. The result of the calculations is displayed on the LCD portion.
The ROM stores, as a constituent element of the program necessary for the computations, a function for determining glucose concentration C in particular. This function is defined as follows. C is expressed by a below-indicated equation (1), where a; (i=0, 1, 2, 3, 4, 5) is determined from a plurality of pieces of measurement data in advance according to the following procedure:

(1) A multiple regression equation is created that indicates the relationship between the normalized parameter and the glucose concentration C.
(2) Normalized equations (simultaneous equations) relating to the normalized parameter are obtained from an equation obtained by the least-squares method.

(3) Values of coefficient $a_i$ (i=0, 1, 2, 3, 4, 5) are determined from the normalized equation and then substituted into the multiple regression equation.

Initially, the regression equation (1) indicating the relationship between the glucose concentration C and the normalized parameters $X_1$, $X_2$, $X_3$, $X_4$ and $X_5$ is formulated.

$$C = f(X_1, X_2, X_3, X_4, X_5)$$
$$= a_0 + a_1 X_1 + a_2 X_2 + a_3 X_3 + a_4 X_4 + a_5 X_5 \quad ......(1)$$

Then, the least-squares method is employed to obtain a multiple regression equation that would minimize the error with respect to a measured value $C_i$ of glucose concentration according to an enzyme electrode method. When the sum of the squares of the residual is D, D is expressed by the following equation (2):

$$D = \sum_{i=1}^{n} d_i^2$$
$$= \sum_{i=1}^{n} (C_i - f(X_{i1}, X_{i2}, X_{i3}, X_{i4}, X_{i5}))^2$$
$$= \sum_{i=1}^{n} \{C_i - (a_0 + a_1 X_{i1} + a_2 X_{i2} + a_3 X_{i3} + a_4 X_{i4} + a_5 X_{i5})\}^2 \quad ......(2)$$

The sum of the squares of the residual D becomes minimum when partial differentiation of equation (2) with respect to $a_0$, $a_2$, ..., $a_5$ gives zero. Thus, we have the following equations:

$$\frac{\partial D}{\partial a_0} = -2\sum_{i=1}^{n} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_1} = -2\sum_{i=1}^{n} X_{i1}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_2} = -2\sum_{i=1}^{n} X_{i2}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_3} = -2\sum_{i=1}^{n} X_{i3}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_4} = -2\sum_{i=1}^{n} X_{i4}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_5} = -2\sum_{i=1}^{n} X_{i5}\{C_i - (a_0 + a_1 X_{i1} + a_2 X_{i2} + a_3 X_{i3} + a_4 X_{i4} + a_5 X_{i5})\} = 0 \quad ......(3)$$

When the mean values of C and $X_1$ to $X_5$ are $C_{mean}$ and $X_{1mean}$ to $X_5$mean, respectively, since $X_{imean}=0$ (i=1 to 5), equation (1) yields equation (4) thus:

$$a_0 = C_{mean} - a_1 X_{1mean} - a_2 X_{2mean} - a_3 X_{3mean} - a_4 X_{4mean} - a_5 X_{5mean}$$
$$= C_{mean} \qquad \qquad ......(4)$$

The variation and covariation between the normalized parameters are expressed by equation (5). Covariation between the normalized parameter $X_i$ (i=1 to 5) and C is expressed by equation (6).

$$S_{ij} = \sum_{k=1}^{n}(X_{ki} - X_{imean})(X_{kj} - X_{jmean}) = \sum_{k=1}^{n} X_{ki} X_{kj} \quad (i, j = 1,2,..5) \quad ......(5)$$

$$S_{iC} = \sum_{k=1}^{n}(X_{ki} - X_{imean})(C_k - C_{mean}) = \sum_{k=1}^{n} X_{ki}(C_k - C_{mean}) \quad (i = 1,2,..5) \quad ......(6)$$

Substituting equations (4), (5), and (6) into equation (3) and rearranging yields simultaneous equations (normalized equations) (7). Solving equations (7) yields $a_1$ to $a_5$.

$$\alpha_1 S_{11} + \alpha_2 S_{12} + \alpha_3 S_{13} + \alpha_4 S_{14} + \alpha_5 S_5 \; S_{1C}$$

$$\alpha_1 S_{21} + \alpha_2 S_{22} + \alpha_3 S_{23} + \alpha_4 S_{24} + \alpha_5 S_{25} = S_{2C}$$

$$\alpha_1 S_{31} + \alpha_2 S_{32} + \alpha_3 S_{33} + \alpha_4 S_{34} + \alpha_5 S_{35} = S_{3C}$$

$$\alpha_1 S_{41} + \alpha_2 S_{42} + \alpha_3 S_{43} + \alpha_4 S_{44} + \alpha_5 S_{45} = S_{4C}$$

$$\alpha_1 S_{51} + \alpha_2 S_{52} + \alpha_3 S_{53} + \alpha_4 S_{54} + \alpha_5 S_{55} \; S_{5C} \qquad (7)$$

Constant term $a_0$ is obtained by means of equation (4). The thus obtained $a_i$ (i=0, 1, 2, 3, 4, 5) is stored in ROM at the time of manufacture of the apparatus. In actual measurement using the apparatus, the normalized parameters $X_1$ to $X_5$ obtained from the measured values are substituted into regression equation (1) to calculate the glucose concentration C.

Hereafter, an example of the process of calculating the glucose concentration will be described. The coefficients in equation (1) are determined in advance based on a large quantity of data obtained from able-bodied persons and diabetic patients. The ROM in the microprocessor stores the following formula for the calculation of glucose concentration:

$$C = 99.4 + 18.3 \times X_1 - 20.2 \times X_2 - 23.7 \times X_3 - 22.0 \times X_4 - 25.9 \times X_5$$

$X_1$ to $X_5$ are the results of normalization of parameters $x_1$ to $x_5$. Assuming the distribution of the parameters is normal, 95% of the normalized parameters take on values between -2 and +2.

In an example of measured values for an able-bodied person, substituting normalized parameters $X_1$=-0.06, $X_2$=+0.04 and $X_3$=+0.05, $X_4$=-0.12 and $X_5$=+0.10 in the above equation yields C=96 mg/dL. In an example of measured values for a diabetic patient, substituting normalized parameters $X_1$=+1.15, $X_2$=-1.02, $X_3$=-0.83, $X_4$=-0.91 and $X_5$=-1.24 in the equation yields C=213 mg/dL.

Hereafter, the results of measurement by the conventional enzymatic electrode method and those by the embodiment of the invention will be described. In the enzymatic electrode method, a blood sample is reacted with a reagent and the amount of resultant electrons is measured to determine the blood sugar level. When the glucose concentration was 89 mg/dL according to the enzymatic electrode method in an example of measured values for an able-bodied person, substituting normalized parameters $X_1$=-0.06, $X_2$=+0.04, $X_3$=+0.05, $X_4$=-0.12 and $X_5$=+0.10 obtained by measurement at the same time according to the inventive method into the above equation yield C=96 mg/dL. Further, when the glucose concentration was 238 mg/dL according to the enzymatic electrode method in an example of measurement values for a diabetic patient, substituting $X_1$=+1.15, $X_2$=-1.02, $X_3$=-0.83, $X_4$=-0.91 and $X_5$=-1.24 obtained by measurement at the same time according to the inventive method yields C=213 mg/dL. From the above results, it has been confirmed that the glucose concentration can be accurately determined using the method of the invention.

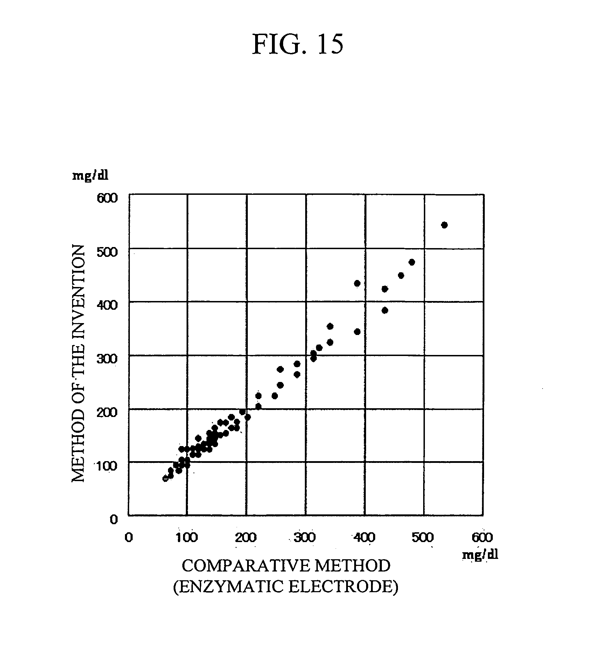

Fig. 15 shows a chart plotting on the vertical axis the values of glucose concentration calculated by the inventive method and on the horizontal axis the values of glucose concentration measured by the enzymatic electrode method, based on a plurality of patients. A good correlation is obtained by measuring the oxygen supply volume and blood flow volume according to the invention (correlation coefficient = 0.9324).

In the above-described embodiment, the parameters relating to blood hemoglobin concentration and blood hemoglobin oxygen saturation have been obtained by spectroscopically measuring the hemoglobin in blood. However, the hemoglobin concentration is stable in persons without such symptoms as anemia, bleeding or erythrocytosis. The hemoglobin concentration is normally in the range between 13 to 18 g/dL for males and between 12 to 17 g/dL for females, and the range of variation of hemoglobin concentration from the normal values is 5 to 6%. Further, the weight of the term relating to the blood flow volume in the aforementioned formula for calculating blood sugar level is smaller than other terms. Therefore, the hemoglobin concentration can be treated as a constant without greatly lowering the measurement accuracy. Similarly, the hemoglobin oxygen saturation is stable between 97 to 98% if the person is undergoing aerial respiration at atmospheric pressure, at rest and in a relaxed state. Thus the hemoglobin concentration and the hemoglobin oxygen saturation can be treated as constants, and the oxygen supply volume can be determined from the product of the hemoglobin concentration constant, the hemoglobin oxygen saturation constant and the blood flow volume.

By treating the hemoglobin concentration and hemoglobin oxygen saturation as constants, the sensor arrangement for measuring blood sugar level can be simplified by removing the optical sensors, for example. Further, by eliminating the time necessary for optical measurement and the processing thereof, the procedure for blood sugar level measurement can be accomplished in less time.

Because the hemoglobin oxygen saturation takes on a stable value when at rest, in particular, by treating the hemoglobin concentration and hemoglobin oxygen saturation as constants, the measurement accuracy for blood sugar level measurement when at rest can be increased, and the procedure for blood sugar level measurement can be accomplished in less time. By "when at rest" herein is meant the state in which the test subject has been either sitting on a chair or lying and thus moving little for approximately five minutes.

Hereafter, an embodiment will be described in which the blood hemoglobin concentration and blood hemoglobin oxygen saturation are treated as constants. This embodiment is similar to the above-described embodiment except that the blood hemoglobin concentration and blood hemoglobin oxygen saturation are treated as constants, and therefore the following description mainly concerns the differences from the earlier embodiment.

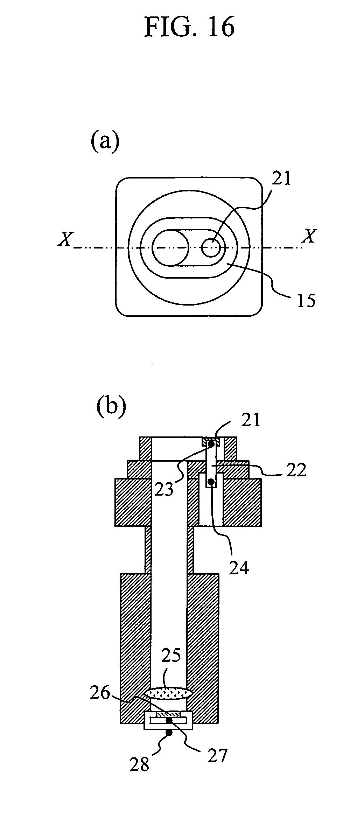

In the present embodiment, the hemoglobin concentration and hemoglobin oxygen saturation shown in Fig. 4 are not measured but treated as constants. Therefore, as shown in Fig. 16, the measurement portion of the present embodiment has the structure of the measurement portion of the earlier embodiment shown in Fig. 13 from which the light sources 33 and 34, photodiode 35 and optical fibers 31 and 32 have been removed. The material and size of the heat-conducting member 22 are the same as those of the previous embodiment, and so is the position where the thermistor 24 comes into contact with the heat-conducting member 22. Parameters used in the present embodiment are parameter $x_1$ proportional to heat radiation, parameter $x_2$ related to heat convection, and parameter $x_3$ proportional to the oxygen supply volume (hereafter, parameter proportional to oxygen supply volume will be indicated as $x_3$). From these parameters, normalized parameters are calculated in the manner described above, and a glucose concentration is calculated based on the three normalized parameters $X_i$ (i=1, 2, 3). During data processing, the step "CONVERSION OF OPTICAL MEASUREMENT DATA INTO NORMALIZED PARAMETERS" (see Fig. 14), which is necessary in the previous embodiment, can be eliminated.

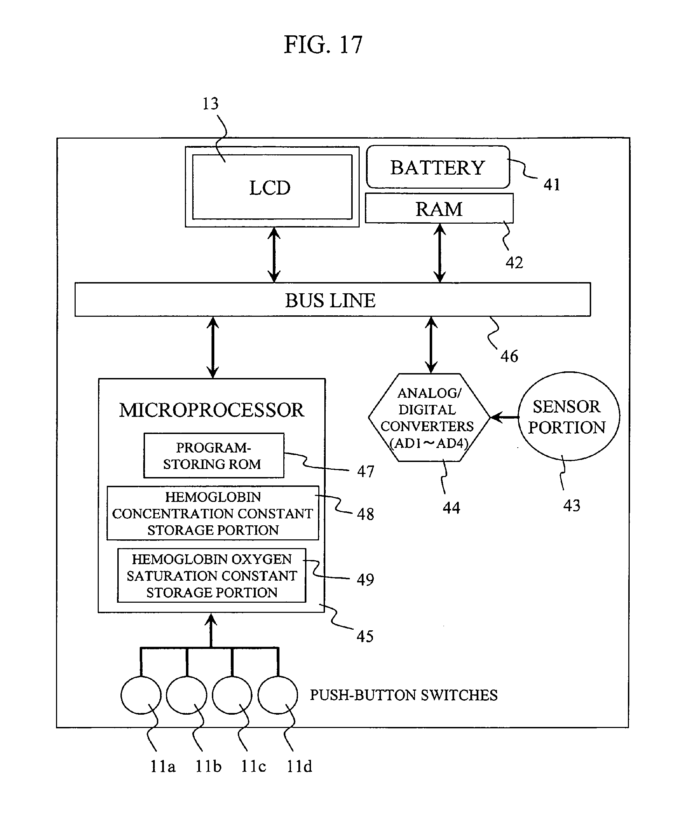

Fig. 17 shows a functional block diagram of the apparatus according to the embodiment. The apparatus runs on battery 41. Signals measured by sensor portion 43 including a temperature sensor are fed to analog/digital converters 44 (AD1 to AD4) provided for individual signals where they are converted into digital signals. Analog/digital converters AD1 to AD4, LCD 13 and RAM 42 are peripheral circuits to microprocessor 45. They are accessed by the microprocessor 45 via bus line 46. The push buttons 11a to 11d are connected to microprocessor 45. The microprocessor 45 includes the ROM for storing software. By pressing the buttons 11a to 11d, external instructions can be entered into microprocessor 45.

The ROM 47 included in the microprocessor 45 stores a program necessary for computations, i.e., it has the function of an arithmetic unit. The microprocessor 45 further includes a hemoglobin concentration constant storage portion 48 for storing hemoglobin concentration constants, and a hemoglobin oxygen saturation constant storage portion 49 for storing hemoglobin oxygen saturation constants. After the measurement of the finger is finished, the computing program calls up optimum constants from the hemoglobin concentration storage portion 48 and hemoglobin oxygen saturation constant storage portion 49 and perform calculations. A memory area necessary for computations is ensured in the RAM 42 similarly incorporated into the apparatus. The result of computations is displayed on the LCD portion.

The ROM stores, as a constituent element of the program necessary for the computations, a function for determining glucose concentration C in particular. The function is defined as follows. C is expressed by a below-indicated equation (8), where $a_i$ (i=0, 1, 2, 3) is determined from a plurality of pieces of measurement data in advance according to the following procedure:

(1) A multiple regression equation is created that indicates the relationship between the normalized parameter and the glucose concentration C.
(2) Normalized equations (simultaneous equations) relating to the normalized parameter are obtained from an equation obtained by the least-squares method.
(3) Values of coefficient a; (i=0, 1, 2, 3) are determined from the normalized equation and then substituted into the multiple regression equation.

Initially, the regression equation (8) indicating the relationship between the glucose concentration C and the normalized parameters $X_1$, $X_2$ and $X_3$ is formulated.

$$C = f(X_1, X_2, X_3)$$
$$= a_0 + a_1 X_1 + a_2 X_2 + a_3 X_3 \quad \ldots\ldots(8)$$

Then, the least-squares method is employed to obtain a multiple regression equation that would minimize the error with respect to a measured value $C_i$ of glucose concentration according to an enzyme electrode method. When the sum of squares of the residual is D, D is expressed by the following equation (9):

$$D = \sum_{i=1}^{n} d_i^{\ 2}$$
$$= \sum_{i=1}^{n} (C_i - f(X_{i1}, X_{i2}, X_{i3}))^2$$
$$= \sum_{i=1}^{n} \{C_i - (a_0 + a_1 X_{i1} + a_2 X_{i2} + a_3 X_{i3})\}^2 \quad \ldots\ldots(9)$$

The sum of squares of the residual D becomes minimum when partial differentiation of equation (9) with respect to $a_0$ to $a_3$ gives zero. Thus, we have the following equations:

$$\frac{\partial D}{\partial a_0} = -2\sum_{i=1}^{n} \{C_i - (a_0 + a_1 X_{i1} + a_2 X_{i2} + a_3 X_{i3})\} = 0$$

$$\frac{\partial D}{\partial a_1} = -2\sum_{i=1}^{n} X_{i1} \{C_i - (a_0 + a_1 X_{i1} + a_2 X_{i2} + a_3 X_{i3})\} = 0$$

$$\frac{\partial D}{\partial a_2} = -2\sum_{i=1}^{n} X_{i2} \{C_i - (a_0 + a_1 X_{i1} + a_2 X_{i2} + a_3 X_{i3})\} = 0$$

$$\frac{\partial D}{\partial a_3} = -2\sum_{i=1}^{n} X_{i3} \{C_i - (a_0 + a_1 X_{i1} + a_2 X_{i2} + a_3 X_{i3})\} = 0 \quad \ldots\ldots(10)$$

When the mean values of C and $X_1$ to $X_3$ are $C_{mean}$ and $X_{1mean}$ to $X_{3mean}$, respectively, since $X_{imean}=0$ (i=1

to 3), equation (8) yields equation (11) thus:

$$a_0 = C_{mean} - a_1 X_{1mean} - a_2 X_{2mean} - a_3 X_{3mean}$$
$$= C_{mean} \qquad \qquad \text{......(11)}$$

The variation and covariation between the normalized parameters are expressed by equation (12). Covariation between the normalized parameter $X_i$ (i=1 to 3) and C is expressed by equation (13).

$$S_{ij} = \sum_{k=1}^{n}(X_{ki} - X_{imean})(X_{kj} - X_{jmean}) = \sum_{k=1}^{n} X_{ki} X_{kj} \quad (i, j = 1,2,3) \quad \text{......(12)}$$

$$S_{iC} = \sum_{k=1}^{n}(X_{ki} - X_{imean})(C_k - C_{mean}) = \sum_{k=1}^{n} X_{ki}(C_k - C_{mean}) \quad (i = 1,2,3) \quad \text{......(13)}$$

Substituting equations (11), (12), and (13) into equation (10) and rearranging yields simultaneous equations (normalized equations) (14). Solving equations (14) yields $a_1$ to $a_3$.

$$\alpha_1 S_{11} + \alpha_2 S_{12} + \alpha_3 S_{13} = S_{1C}$$

$$\alpha_1 S_{21} + \alpha_2 S_{22} + \alpha_3 S_{23} = S_{2C}$$

$$\alpha_1 S_{31} + \alpha_2 S_{32} + \alpha_3 S_{33} = S_{3C} \qquad \qquad (14)$$

Constant term $a_0$ is obtained by means of equation (11). The thus obtained $a_i$ (i=0, 1, 2, 3) is stored in ROM at the time of manufacture of the apparatus. In actual measurement using the apparatus, the normalized parameters $X_1$ to $X_3$ obtained from the measured values are substituted into regression equation (8) to calculate the glucose concentration C.

Hereafter, an example of the process of calculating the glucose concentration will be described. The coefficients in equation (8) are determined in advance based on a large quantity of data obtained from able-bodied persons and diabetic patients. The ROM in the microprocessor stores the following formula for the calculation of glucose concentration:

$$C = 101.7 + 25.8 \times X_1 - 23.2 \times X_2 - 12.9 \times X_3$$

$X_1$ to $X_3$ are the results of normalization of parameters $x_1$ to $x_3$. Assuming the distribution of the parameters is normal, 95% of the normalized parameters take on values between -2 and +2.

In an example of measured values for an able-bodied person, substituting normalized parameters $X_1$=-0.06, $X_2$=+0.04 and $X_3$=+0.10 in the above equation yields C=101 mg/dL. In an example of measured values for a diabetic patient, substituting normalized parameters $X_1$=+1.35, $X_2$=-1.22 and $X_3$=-1.24 in the equation yields C=181 mg/dL. In the above equation, the hemoglobin concentration and hemoglobin oxygen saturation are rendered into constants of 15 g/dL and 97%, respectively.

Hereafter, the results of measurement by the conventional enzymatic electrode method and those by the embodiment of the invention will be described. In the enzymatic electrode method, a blood sample is reacted with a reagent and the amount of resultant electrons is measured to determine glucose concentration. When the glucose concentration was 93 mg/dL according to the enzymatic electrode method in an example of measured values for an able-bodied person, substituting normalized parameters $X_1$=-0.06, $X_2$=+0.04 and $X_3$=+0.10 obtained by measurement at the same time according to the inventive method into the above equation yielded C=101 mg/dL. Further, when the glucose concentration was 208 mg/dL according to the enzymatic electrode method in an example of measurement values for a diabetic patient, substituting $X_1$=+1.35, $X_2$=-1.22 and $X_3$=-1.24 obtained by measurement at the same time according to the inventive method yielded C=181 mg/dL. Although the calculation results indicate an error of about 13%, this level of accuracy is considered sufficient because normally errors between 15% and 20% are considered acceptable in blood sugar level measuring apparatuses in general. Thus, it has been confirmed that the method of the invention can allow glucose concentrations to be determined with high accuracy.

Fig. 21 shows a chart plotting on the vertical axis the values of glucose concentration calculated by the inventive method and on the horizontal axis the values of glucose concentration measured by the enzymatic electrode method, based on measurement values obtained from a plurality of patients. A good correlation is obtained by measuring according to the invention (correlation coefficient = 0.8932).

## Claims

1.  A blood sugar level measuring apparatus comprising:

    a heat amount measurement portion for measuring a plurality of temperatures deriving from a body surface and obtaining information used for calculating the amount of heat transferred by convection and the amount of heat transferred by radiation, both related to the dissipation of heat from said body surface;
    an oxygen amount measurement portion for obtaining information about the blood oxygen amount;
    a memory portion for storing relationships between parameters corresponding to said plurality of temperatures and blood oxygen amount and blood sugar levels;
    a calculating portion which converts a plurality of measurement values fed from said heat amount measuring portion and said oxygen amount measurement portion into said parameters, and computes a blood sugar level by applying said parameters to said relationships stored in said storage portion; and
    a display portion for displaying the result calculated by said calculating portion,

    wherein:

    said oxygen amount measurement portion includes a blood flow volume measurement portion for obtaining information about the blood flow volume, and an optical measurement portion for obtaining the hemoglobin concentration and hemoglobin oxygen saturation in blood,

    wherein said blood flow volume measurement portion includes:

    a body-surface contact portion;
    a first temperature detector disposed adjacent to said body-surface contact portion;
    a heat-conducting member disposed adjacent to said body-surface contact portion; and
    a second temperature detector for detecting the temperature at a position on said heat-conducting member that is spaced apart from said body-contact potion by 3.6 mm or more.

2.  A blood sugar level measuring apparatus comprising:

    an ambient temperature measuring portion for measuring ambient temperature;
    a body-surface contact portion to be brought into contact with a body surface;
    an adjacent temperature detector disposed adjacent to said body-surface contact portion;
    a radiation heat detector for measuring radiation heat from said body surface;
    a heat-conducting member disposed adjacent to said body-surface contact portion;
    an indirect temperature detector disposed adjacent to said heat-conducting member at a position spaced apart from said body-surface contact portion by 3.6 mm or more, for detecting the temperature at the position spaced apart from said body-surface contact portion;
    a light source for irradiating said body-surface contact portion with light of at least two different wavelengths;
    a photodetector for detecting reflected light produced as said light of at least two different wavelengths is reflected on said body surface;
    a calculating portion including a conversion portion for converting the outputs of said adjacent temperature detector, said indirect temperature detector, said ambient temperature measuring portion, said radiation heat detector, and said photodetector into parameters, and a processing portion in which relationships between said parameters and blood sugar levels are stored in advance, said processing portion calculating a blood sugar level by applying said parameters to said relationships; and
a display portion for displaying the result outputted from said calculation portion.

3. A blood sugar level measuring apparatus comprising:

   a plate to be brought into contact with a body surface;
   a first temperature sensor for detecting the temperature of said plate;
   a member disposed adjacent to said plate;
   a second temperature sensor disposed on said member at a position spaced apart from said plate by 3.6 mm or more;
   a heat detector for measuring radiation heat from said body surface;
   a light source for irradiating said plate with light;
   a photodetector for detecting light following the irradiation of said body surface;
   a calculation portion for calculating a blood sugar level based on the outputs of said first temperature sensor, said second temperature sensor, said heat detector, and said photodetector.

4. A blood sugar level measuring apparatus comprising:

   an ambient temperature measuring portion for measuring ambient temperature;
   a body-surface contact portion to be brought into contact with a body surface;
   an adjacent temperature detector disposed adjacent to said body-surface contact portion;
   a radiation heat detector for measuring radiation heat from said body surface;
   a heat-conducting member disposed adjacent to said body-surface contact portion;
   an indirect temperature detector disposed adjacent to said heat-conducting member at a position spaced apart from said body-surface contact portion by 3.6 mm or more, for detecting the temperature at the position spaced apart from said body-surface contact portion;
   a memory portion in which information regarding blood hemoglobin concentration and hemoglobin oxygen saturation is stored;
   a calculation portion including a conversion portion for converting the outputs of said adjacent temperature detector, said indirect temperature detector, said ambient temperature measuring portion, and said radiation heat detector, into a plurality of parameters, and a processing portion in which relationships between said parameters and blood sugar levels are stored, said calculation portion calculating a blood sugar level by applying said parameters to said relationships; and
   a display portion for displaying the result outputted from said calculation portion.

5. A blood sugar level measuring apparatus according to any preceding claim, wherein said member has a length of 3.6 mm or more.

6. A blood sugar level measuring apparatus according to any preceding claim, wherein said member has a heat conductivity in the range of from 0.1 J/s·m·K to 0.3 J/s·m·K.

7. A blood sugar level measuring apparatus according to any preceding claim, wherein said member is made of polyvinylchloride or ABS resin.

FIG. 1

ROOM
TEMPERATURE ($T_r$)

BLOCK

HEAT
DISSIPATION $T_2$

HEAT
TRANSFER

BODY-
SURFACE
TEMPERATURE ($T_s$) $T_1$

BLOOD FLOW
VOLUME

CAPILLARY BLOOD VESSEL

EP 1 595 493 A1

## FIG. 2

## FIG. 3

2/15

21

# FIG. 4

FIG. 5

## FIG. 6

SKIN TEMPERATURE(° C)

BLOOD FLOW VOLUME (ml/100ml•min)

## FIG. 7

HEAT CONDUCTIVITY (cal/cm•s•k)

BLOOD FLOW VOLUME (ml/100ml•min)

# FIG. 8

TEMPERATURE DISTRIBUTION AT TIME t1

TEMPERATURE

TEMPERATURE DISTRIBUTION AT TIME t2

TEMPERATURE DISTRIBUTION AT TIME t3

BLOCK

POSITION(x)

x=0
CONTACT
POSITION

x1
TEMPERATURE PERMEATION
THICKNESS POSITION
AT TIME t1

x2
TEMPERATURE PERMEATION
THICKNESS POSITION
AT TIME t2

# FIG. 9

TEMPERATURE PERMEATION
THICKNESS(m)

MEASUREMENT TIME(s)

# FIG. 10

MOVING SPEED OF TEMPERATURE
PERMEATION THICKNESS(m/√s)

MEASUREMENT TIME(s)

FIG. 11

95 mg/dl

# FIG. 12

```
        ╭─────────────╮
        │    START    │
        ╰──────┬──────╯
               ▼
        ┌─────────────┐
        │ CIRCUIT TEST│
        └──────┬──────┘
               ▼
        ┌─────────────┐
        │ WARMING UP  │
        └──────┬──────┘
               ▼
        ┌─────────────┐
        │PLACE FINGER │
        └──────┬──────┘
               ▼
        ┌─────────────┐
        │  COUNTDOWN  │
        └──────┬──────┘
               ▼
        ┌─────────────┐
        │ LIFT FINGER │
        └──────┬──────┘
               ▼
        ┌─────────────┐
        │DATA PROCESSING│
        └──────┬──────┘
               ▼
        ┌─────────────┐
        │DISPLAY OF DATA│
        │  95  mg/dl  │
        └──────┬──────┘
               ▼
        ╭─────────────╮
        │    END      │
        ╰─────────────╯
```

# FIG. 13

(a)

(b)

(c)

FIG. 14

# FIG. 15

# FIG. 16

(a)

(b)

FIG. 17

LCD

13

BATTERY

41

RAM

42

BUS LINE

46

MICROPROCESSOR

PROGRAM-
STORING ROM

47

HEMOGLOBIN
CONCENTRATION CONSTANT
STORAGE PORTION

48

HEMOGLOBIN OXYGEN
SATURATION CONSTANT
STORAGE PORTION

49

45

ANALOG/
DIGITAL
CONVERTERS
(AD1~AD4)

44

SENSOR
PORTION

43

PUSH-BUTTON SWITCHES

11a  11b  11c  11d

FIG. 18

European Patent
Office

**EUROPEAN SEARCH REPORT**

Application Number

EP 04 02 9345

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 01/28414 A (KAUFMANN-KIM, YUN-OAK; CHO, OK-KYUNG) 26 April 2001 (2001-04-26)<br>* page 9, line 10 - line 25 *<br>* page 11, line 15 - page 12, line 25 *<br>* page 19, line 32 - page 20, line 7 *<br>----- | 1-4 | A61B5/00 |
| A,D | US 5 795 305 A (CHO ET AL)<br>18 August 1998 (1998-08-18)<br>* column 6, line 30 - column 7, line 27 *<br>----- | 1-4 | |
| P,A | JP 03 566278 B1 (HITACHI, LTD)<br>15 September 2004 (2004-09-15)<br>* the whole document * | 1-7 | |
| E | -& EP 1 495 714 A (HITACHI, LTD)<br>12 January 2005 (2005-01-12)<br>----- | 1-7 | |
| | | | TECHNICAL FIELDS SEARCHED (Int.Cl.7)<br><br>A61B |

The present search report has been drawn up for all claims

| Place of search | Date of completion of the search | Examiner |
|---|---|---|
| Munich | 22 March 2005 | Alvazzi Delfrate, S |

EPO FORM 1503 03.82 (P04C01)

**ANNEX TO THE EUROPEAN SEARCH REPORT
ON EUROPEAN PATENT APPLICATION NO.**                    EP 04 02 9345

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2005

| Patent document cited in search report | | Publication date | Patent family member(s) | | Publication date |
|---|---|---|---|---|---|
| WO 0128414 | A | 26-04-2001 | WO | 0128414 A2 | 26-04-2001 |
| US 5795305 | A | 18-08-1998 | DE | 4342105 A1 | 14-06-1995 |
| | | | WO | 9515711 A1 | 15-06-1995 |
| | | | EP | 0734222 A1 | 02-10-1996 |
| | | | JP | 9509584 T | 30-09-1997 |
| JP 3566278 | B1 | 15-09-2004 | EP | 1495714 A1 | 12-01-2005 |
| | | | US | 2005010094 A1 | 13-01-2005 |
| EP 1495714 | A | 12-01-2005 | JP | 3566278 B1 | 15-09-2004 |
| | | | EP | 1495714 A1 | 12-01-2005 |
| | | | US | 2005010094 A1 | 13-01-2005 |

EPO FORM P0459

For more details about this annex : see Official Journal of the European Patent Office, No. 12/82